# DEMANDE DE BREVET EUROPEEN

(11) **EP 3 785 709 A1**
(43) Date de publication de la demande: **03.03.2021**
(21) Numéro de dépôt: 20020390.9
(22) Date de dépôt: 28.08.2020
(51) Int. Cl.: A61K 31/015, A61K 31/105, A61K 31/11, A61K 31/255, A61K 31/56, A61K 31/575, A61P 31/18

(54) **COMPOSITION PHARMACEUTIQUE DESTINÉE À INHIBER L'INFECTIOSITÉ DU VIH, À TRAITER LE SYNDROME D'IMMUNODÉFICIENCE ACQUISE (SIDA) ET SES COMPLICATIONS**

(30) Priorité: 30.08.2019 FR 1909590
(71) Demandeur: Monkam Nitcheu, Guy Faustin, 59800 Lille (FR)
(72) Inventeur: Monkam Nitcheu, Guy Faustin, 59800 Lille (FR)

(57) **Abrégé**

La présente invention concerne une composition pharmaceutique utilisée pour inhiber la pénétration d'agents pathogènes dans les cellules cibles. Elle peut être également utilisée dans la prévention ou le traitement d'infections par le VIH, empêche l'apparition ou la progression du sida et ses différentes conséquences.

## Description

La présente invention concerne une composition pharmaceutique qui peut être utilisée pour le traitement thérapeutique d'une infection par un agent pathogène possédant une enveloppe bicouche lipidique, notamment le virus de l'immunodéficience acquise (VIH), et de ses complications.

### Art Anterieur

L'AZT, initialement conçu pour bloquer la multiplication des cellules cancéreuses, s'est révélé à la fois inefficace et toxique. En revanche, sa capacité à bloquer un des mécanismes de la réplication du VIH en a fait le premier médicament contre le syndrome d'immunodéficience acquise (SIDA). Au cours des dernières décennies, des progrès remarquables ont été réalisés dans la prévention contre cette pandémie. Cette prévention implique une stratégie intégrée qui articule de manière combinée la prévention primaire, le dépistage et la prise en charge des personnes séropositives.

D'autre part, l'amélioration des connaissances des processus d'infection par le virus de l'immunodéficience humaine (VIH) a permis l'identification de nouvelles cibles et la mise au point des traitements antirétroviraux hautement actifs (HAART). Ces traitements perturbent le cycle de réplication du VIH et visent à diminuer de façon profonde et durable la quantité de virus circulant dans l'organisme, et surtout de faire remonter le nombre de lymphocytes T auxiliaires.

Ces antirétroviraux sont des molécules de synthèses de différentes natures chimiques. Ils se subdivisent en quatre grandes familles : les inhibiteurs nucléosidiques ou non nucléosidiques de la transcriptase inverse, les inhibiteurs de protéase, les inhibiteurs de fusion et les inhibiteurs d'intégrase.
- Les inhibiteurs nucléosidiques de la transcriptase inverse, sont les premiers antirétroviraux développés actifs sur le VIH-1 et -2. Ils rentrent en compétition avec les substrats naturels de la transcriptase inverse et bloquent la fabrication de l'ADN pro-viral.
- Les inhibiteurs non nucléosidiques de la transcriptase inverse, agissent sans interférer sur le substrat de l'enzyme. Ils sont spécifiques le la transcriptase inverse du VIH-1 et inactif sur le VIH-2.
- Les inhibiteurs de protéase sont tous des peptidomimétiques, ils agissent au niveau du processus d'assemblage des protéines virales nouvellement synthétisées. Ainsi, en se fixant sur le site actif de la protéase virale, ils l'empêchent de catalyser le clivage des polypeptides du virus. La maturation protéique étant altérée, les virions formés sont inactifs et non infectieux.
- Les inhibiteurs de fusion, empêchent le VIH de s'attacher à la surface externe des cellules, et ainsi, d'entrer dans ces dernières.
- Les inhibiteurs de l'intégrase, bloquent l'intégration de l'ADN pro-viral au génome de la cellule infectée.

De nouvelles stratégies de traitement contre le VIH sont ainsi apparues : Il s'agit d'associations utilisées à différents fins, que ce soit dans le cadre d'une combinaison de médicaments de première intention, en l'occurence la trithérapie ou encore d'associations plus complexes ciblant les personnes déjà traitées, ayant une accumulation de résistances, et justifiant ainsi le passage à une quadrithérapie voir à une pentathérapie. Par rapport à une monothérapie utilisant l'AZT, ces thérapies combinées antirétrovirales se sont révélées capables de supprimer efficacement la réplication virale. Cependant elles ne permettent qu'une régénération partielle du système immunitaire, et la réponse immunitaire T spécifique du VIH n'est pas totalement restaurée.

D'autre part, ces combinaisons thérapeutiques antivirales ne guérissent pas de l'infection par le VIH et aucun des vaccins développés à base de vecteurs viraux ou bactériens, de pseudo-virions ou de peptides ne protège de l'infection consécutive à une épreuve expérimentale. Tout arrêt du traitement est suivi d'une multiplication des virus qui persistent dans l'organisme à l'état latent dans les cellules T mémoire, et du développement des maladies opportunistes (tuberculose, septicémies, infections dues aux champignons, infections parasitaires, infections virales provoquant l'herpes génital, le zona, la leucoencéphalopathie multifocale progressive) caractéristiques du stade sida chez les patients séropositifs. De plus ces médicaments sont responsables d'effets secondaires importants, souvent transitoires, mais aussi à long terme. On y retrouvent : les troubles physiques (modifications corporelles, fatigue, ballonnements, nausées, vomissements, perte d'appétit, fièvres, diarrhées, réactions cutanées...), les troubles neuropsychiques (troubles du sommeil, sentiment de vide, tristesse, perte de mémoire, crampes, douleurs au niveau des muscles...), altération de la qualité de vie (limitation due à la fatigue, gêne liée aux modifications corporelles, aux troubles neuropsychiques), anomalies hématologiques (anémie, lymphopénie, leucopénie, thrombopénie, neutropénie), une hépatotoxicité plus fréquente chez les patients co-infectés, une lipodystrophie localisée ou généralisée, une dyslipidémie pouvant s'accompagner d'un diabète, de maladies cardiovasculaires, d'athérosclérose, d'artériopathie périphérique ou artériopathie oblitérante des membres inférieures, de maladies thromboemboliques, des lésions rénales, des anomalies osseuses.

Par ailleurs, la prévalence de la co-infection par le virus de l'hépatite B (VHB) ou le virus de l'hépatite C (VHC) chez les personnes vivants avec le VIH est élevée, ceci à cause des modes de contamination similaires. Ces co-infections aboutissent à de nombreuses recombinaisons entre souches de virus d'ou émergent des souches mosaïques dites formes recombinantes en circulation (CRF : Circulating recombinant forms). De plus, de nombreux médicaments anti-VIH sont dégradés par le foie, ce qui peut stresser cet organe et lui causer davantage de dommages. La prise en charge thérapeutique de l'infection à VHB ou VHC est limitée et prend en compte la nécessité de traiter ou non l'infection par le VIH, d'intégrer les traitements anti-VHB ou anti-VHC et anti-VIH au sein d'une stratégie globale permettent d'éviter la chronicité de l'infection, de régresser la fibrose, de prévenir les complications de la cirrhose et la survenue d'un carcinome hépatocellulaire. En effet, ces différents traitements interagissent les uns avec les autres et peuvent causer des effets secondaires plus nombreux ou plus grave, et impose de ce fait des contraintes de surveillance virologique, immunologique, biochimique et histologique.

D'autre part, le vieillissement des patients vivants avec le VIH a conduit à l'émergence de comorbidité et d'autres causes de mortalité, notamment les maladies cardiovasculaires, les cancers en particulier les cancers classant sida (le sarcome de kaposi, les lymphomes, le cancer du col de l'utérus, le cancer du foie...etc), qui représentent un nouveau défi dans la prise en charge des personnes traitées par les antirétroviraux en succès sérologique. L'immunodépression cellulaire induite par le VIH favoriserait la persistance et l'oncogénicité des agents pathogènes. De plus, la chimiothérapie conventionnelle cytotoxique ciblant autant les cellules tumorales que les cellules de la lignée médullaire constitue un risque important sur terrain fortement déprimé.

Dans ce contexte, la membrane des cellules joue un rôle prépondérant dans un grand nombre de processus physiologiques ou physiopathologiques, et notamment dans l'infection. En effet, elle comporte la plupart des éléments essentiels aux échanges entre la cellule et son environnement. Elle est constituée de micro-domaines encore appelés rafts. Ces derniers sont constitués d'un assemblage compacte de lipides (glycosphingolipides et/ou de la sphingomyéline et du cholestérol) et des protéines (protéines ancrées au glycosylphosphatidylinositol, des protéines liées au cholestérol, des protéines transmembranaires, des tyrosines kinases doublement acétylées de la famille Src, des sous-unités alpha des protéines G hétérotrimériques). Le cholestérol jouant un rôle de «colle» dynamique qui rigidifie, structure ces micro-domaines. Ces protéines, notamment les récepteurs comme celui de l'insuline, les IGF1, les récepteurs hybrides IR/IGF1R, les TLR, les co-récepteurs (CCR5, CXCR4) et bien d'autres encore ont un rôle déterminant dans la signalisation cellulaire, et sont impliquées dans certains processus inflammatoires, infectieux, d'installation des troubles du métabolisme et de cancérogenèse. D'autre part, le dysfonctionnement ou les modifications structurelles qui accompagnent l'activation de cette membrane par des agents pathogènes ou des signaux endogènes peuvent être à l'origine d'une surproduction de microparticules qui contribuent à amplifier l'inflammation, l'hyperactivation immunitaire systémique, la susceptibilité à la coagulation, l'initiation, la progression et l'aggravation de pathologies cardiovasculaires notamment, l'athérosclérose, l'infarctus du myocarde, l'accident vasculaire cérébral et le cancer.

L'inflammation virale expliquerait une bonne part importante des troubles du syndrome métabolique, notamment chez les sujets infectés par le VIH. Elle serait non seulement liée à l'accumulation des lipides dans les cellules, mais aussi à la porosité de la muqueuse intestinale détériorée par le VIH qui permet le passage des bactéries de lumière intestinale vers la circulation sanguine.

Au cour du processus athérosclérotique, l'inflammation artérielle est responsable de l'accumulation sous-endothéliale des lipoprotéines, notamment le cholestérol, ce qui entraine une augmentation de la libération de cytokines pro-inflammatoires, des facteurs de croissance, l'expression accrue de molécules d'adhésion, l'accumulation de cellules apoptotiques, et l'infiltration des macrophages qui à leur tour produisent des médiateurs pro-inflammatoires.

Dans l'inflammation chronique de bas bruit, la production de cytokines pro-inflammatoires sont impliquées dans la pathogenèse du diabète de type 2.

L'inflammation est aussi l'un des symptômes des maladies auto-immunes, qui se caractérisent par un dysfonctionnement du système immunitaire provoqué par une exposition à des substances dangereuses ou toxiques, par une infection notamment virale, par la senescence des cellules immunitaires ou encore par l'âge.

Dans les maladies neurodégénératives, notamment dans la maladie d'Alzheimer, les réactions inflammatoires de défense causées par les cellules immunitaires (macrophages, cellules microgliales, etc) autour de ces plaques séniles contribueraient à la progression de la maladie.

Ces données soulèvent la question du ciblage pharmacologique des membranes des cellules dans la prévention et le traitement de l'infection par le VIH, des maladies qui se caractérisent par une inflammation chronique.

Par ailleurs, le beta-elemene a des propriétés anticancéreuses à large spectre. Il a des propriétés anti-inflammatoires. Ses propriétés immunostimulatrices sont connues. Il améliore l'immunité et la qualité de vie des patients.

Le d-limonene est connu pour son potentiel antiseptique, antiviral. Il possède aussi des propriétés antidiabétiques et hypolipémiantes, et peut à cet effet être considéré comme un agent potentiel pour prévenir et traiter les troubles métaboliques classiquement rencontrés dans les maladies infectieuses. Ses propriétés antioxydantes, anti-inflammatoires et anticancéreuses sont connues. Chez l'homme, le d-limonène a démontré une faible toxicité après une dose unique et répétée pendant un an.

Le beta-sitostérol et le lupéol sont des phytostérols ayant une structure moléculaire similaire à celle du cholestérol. A cet effet, ils peuvent entrer en compétition avec ce dernier. Leurs rôles dans les plantes sont identiques à celle du cholestérol chez l'homme, à savoir maintenir la structure et la fonction de la membrane cellulaire. De nombreuses données scientifiques ont montré que ces stérols possèdent des propriétés hypoglycémiantes, hypolipémiantes, anti-inflammatoires, antipyrétiques et anticancéreuses. Ils ont également des propriétés anti-protozoaires, antimicrobiennes à large spectre. Leur potentiel pharmacologique en fait d'eux, des agents thérapeutiques dans les troubles du syndrome métabolique et des maladies opportunistes au stade sida.

Le cinnamaldéhyde est connu pour ses propriétés hypoglycémiantes, hypolipémiantes et anticancéreuses. Ses propriétés antibactériennes, antifongiques, et antivirales sont connues.

L'Allicine est connu pour ses propriétés anti-inflammatoires, antioxydantes, anticancéreuses, antibactériennes et antivirales. Il permet de prévenir les maladies cardiovasculaires, en particulier en fluidifiant le sang et en luttant contre l'excès de cholestérol et l'hypertension artérielle.

L'ajoene, un des dérivès de l'allicine, est connu pour ses propriétés antioxydants, antithrombotique, anticancéreuse, antibactérienne à large spectre (bactéricide et antifongique) et antivirale.

Le kaempférol est connu pour avoir une bonne activité anti-oxydante. Il augmente le stress des cellules cancéreuses tout en préservant les cellules saines. Il est également connu pour moduler un certain nombre d'éléments clés dans les voies de signalisation impliquées dans l'apoptose, l'angiogenèse, l'inflammation et la métastase.

### [Problème Technique a résoudre]

Un but de la présente invention est de proposer une nouvelle composition pharmaceutique utilisable en tant que médicament et plus particulièrement utilisable dans le traitement du syndrome d'immunodéficience acquise, du syndrome d'immunodéficience de l'âge adulte ou déficit immunitaire combinée sévère.

Un autre but de l'invention est de proposer une nouvelle composition pharmaceutique utilisable en tant que médicament et plus particulièrement utilisable dans la prévention et le traitement du syndrome de l'immunodéficience acquise qui remédie à tout ou une partie des inconvénients liés aux compositions de l'art antérieur précité.

Un autre but de la présente invention est de proposer une composition pharmaceutique qui diminue voir inhibe la réplication virale associée à un état pro-inflammatoire des cellules.

Un autre but de la présente invention est de proposer une composition pharmaceutique utilisable en tant que médicament dans le traitement et/ou la prévention des infections par agent pathogène.

Un autre but de la présente invention est de proposer une composition pharmaceutique qui permet d'inhiber l'entrée du VIH dans la cellule cible, d'induire une immunité humorale neutralisante, une immunité cellulaire vigoureuse au niveau des muqueuses, notamment au niveau du GALT (tissus lymphoïde associé au tube digestif).

Un autre but de l'invention est de proposer une composition pharmaceutique qui permet de bloquer l'infectivité des différentes souches virales sauvages même en dépit de leur énorme variabilité génétique et de cibler les cellules réservoirs qui abritent le virus (formes latentes) dans les organismes des patients sous traitement antirétroviral.

Un autre but de la présente invention est de diminuer voire inhiber l'enrichissement des cellules en cholestérol, l'externalisation des lipides notamment le phosphatidylsérine dans le feuillet externe de la membrane plasmique et la formation des microparticules.

Un autre but de la présente invention est de proposer une composition pharmaceutique qui déstructure, restructure la composition la composition lipidique de la membrane cellulaire, et en conséquence inhibe la pénétration de pénétration de l'agent pathogène dans les cellules, les voies de signalisation cellulaire impliquées dans la réplication virale, la sécrétion des cytokines pro-inflammatoires, la prolifération cellulaire, l'angiogenèse et la métastase.

Un autre but de la présente invention est de proposer une composition pharmaceutique qui permet de réduire voire inhiber les troubles du syndrome métabolique, de traiter le diabète, les dyslipidémies, les maladies cardiovasculaires, le cancer, le syndrome cachectique, le processus de vieillissement y compris la senescence des cellules immunitaires.

Un autre but de la présente invention est de proposer une composition pharmaceutique utilisable dans le traitement et la prévention des tumeurs malignes fréquemment associées à l'immunodépression induite par le VIH, notamment les cancers « classant sida», à savoir, le sarcome de Kaposi, les lymphomes malins non Hodgkiniens (LMNH), le cancer du col de l'utérus, et les cancers « classant non sida ».

Un autre but de la présente invention est de proposer une composition pharmaceutique utilisable en tant que médicament notamment dans le traitement d'une infection par agent pathogène possédant une enveloppe bicouche lipidique.

Un autre but de la présente invention est de proposer une composition pharmaceutique notamment telle que précitée, qui présente une toxicité réduite et/ou qui est bien tolérée par les patients.

### [Brève Description de l'invention]

Pour résoudre au moins un des problèmes techniques précités, la présente invention propose une composition pharmaceutique, qui de manière caractéristique selon l'invention, comprend en tant que principe actif, une combinaison de beta-elemene, de d-limonène, de lupéol, de beta-sitostérol, de cinnamaldéhyde, de l'allicine, de l'ajoéne et eventuellement du kaempférol et leurs mélanges.

Le demandeur a en effet constaté qu'une telle composition pharmaceutique s'avérait active dans la prévention et le traitement du syndrome d'immunodéficience acquise, notamment en retardant la propagation du virus dans l'organisme et en réduisant le nombre d'affections opportunistes ainsi que leur gravité.

Le demandeur a également mis en évidence un effet synergique d'au moins trois des constituants qui procure une action renforcée de la composition sur au moins un mécanisme impliqué dans l'inflammation chronique à savoir un mécanisme choisi parmi, l'accumulation des lipides dans les cellules, la microvésiculation des membranes des cellules, la formation des microparticules, l'hyperactivation des voies de signalisation cellulaire, la surexpression des cytokines pro-inflammatoires, la dégradation autophagique, la pénétration des agents pathogènes dans les cellules.

Cette composition pharmaceutique gripperait le récepteur CD4+ et/ou le (les) co-récepteur(s) CCR5, CXCR4, et empêcherait de ce fait la formation du complexe gp 120-CD4+-CCR5/CXCR4 et la fusion proprement dite entre les membranes virales et cellulaires.

### [Description détaillée]

La composition pharmaceutique selon l'invention peut être utilisée en tant que médicament et notamment pour son utilisation dans le traitement du syndrome d'immunodéficience acquise, du syndrome d'immunodéficience de l'âge adulte ou déficit immunitaire combinée sévère. Selon un mode de réalisation particulier de la présente invention, la composition de l'invention peut comprendre, en outre un mélange de beta-sitostérol et de cinnamaldéhyde ou un mélange de béta-sitostérol et de kaempférol ou un mélange de béta-sitostérol et d'allicine et/ou d'ajoène.

A titre d'exemple, elle peut comprendre en pourcentage massique de la masse totale des ingrédients actifs, un pourcentage de béta-élémène sensiblement égal ou supérieur à 10% et sensiblement égal ou inférieur à 50%, et notamment sensiblement égal ou supérieur à 20% et sensiblement égal ou inférieur à 30%, un pourcentage massique de d-limonene sensiblement égal ou supérieur à 10% et sensiblement égal ou inférieur à 55%, notamment sensiblement égal ou supérieur 20% et sensiblement égal ou inférieur à 40%, un pourcentage massique de lupéol ou un de ses sels pharmaceutiquement acceptables sensiblement égal ou supérieur à 10% et sensiblement égal ou inférieur à 50, et notamment sensiblement égal ou supérieur à 20% et sensiblement égal ou inférieur à 30%, un pourcentage de beta-sitostérol sensiblement égal ou supérieur à 10% et sensiblement égal ou inférieur à 50%, et notamment sensiblement égal ou supérieur à 20% et sensiblement égal ou inférieur à 30%, un pourcentage de cinnamaldéhyde sensiblement égal ou supérieur à 10% et sensiblement égal ou inférieur à 50%, et notamment sensiblement égal ou supérieur à 20% et sensiblement égal ou inférieur à 40%, un pourcentage d'allicine, lorsque ladite composition contient cet ingrédient, sensiblement égal ou supérieur à 10% et sensiblement égal ou inférieur à 50%, et notamment sensiblement égal ou supérieur à 20% et sensiblement égal ou inférieur à 40%, un pourcentage d'ajoène lorsque ladite composition contient cet ingrédient, sensiblement égal ou supérieur à 10% et sensiblement égal ou inférieur à 50%, et notamment sensiblement égal ou supérieur à 20% et sensiblement égal ou inférieur à 40%, un pourcentage de kaempférol lorsque ladite composition contient cet ingrédient, sensiblement égal ou supérieur à 10% et sensiblement égal ou inférieur à 50%, et notamment sensiblement égal ou supérieur à 20% et sensiblement égal ou inférieur à 40%.

Lorsque la composition comprend le beta-elemene et d-limonene, leur pourcentage massique par rapport à la masse totale des ingrédients actifs est notamment égal et notamment sensiblement égal à 40%.

La composition selon l'invention comprend en outre, au moins un excipient pharmaceuticalement acceptable. Cet excipient peut être solide ou liquide. Il peut être choisi, par exemple, parmi l'eau purifiée, l'alcool éthylique, le propylène glycol, la glycérine, les huiles végétales, les huiles animales, les hydrocarbures, les silicones, les sucres tels que le glucose, le levulose, l'amidon de blé, l'amidon de maïs, l'amidon de pomme de terre, la gomme xanthane, la gomme arabique, la gomme adragante, la gomme de Sterculia, la gomme Guar ou "Guaranates", les pectines, les alginates, les carraghénates, la gélose ou Agar-Agar, la gélatine, la cellulose et ses dérivés.

La composition de l'invention peut être administrée par n'importe quelle voie appropriée, par exemple par la voie orale, rectale, locale (topique, par exemple), intrapéritonéale, systémique, intraveineuse, intramusculaire, sous-cutanée ou mucosale, notamment sublinguale, ou bien en utilisant un patch, ou encore sous forme encapsulée dans, ou immobilisée sur, des liposomes, des microparticules, des microcapsules, associée à des nanoparticules et analogues. On peut notamment citer, à titre d'exemples non limitatifs d'excipients appropriés pour une administration par voie orale, le talc, le lactose, l'amidon et ses dérivés, la cellulose et ses dérivés, les polyéthylène glycols, les polymères d'acide acrylique, la gélatine, le stéarate de magnésium, des matières grasses animales, végétales ou synthétiques, les dérivés de la paraffine, les glycols, les stabilisants, les conservateurs, les antioxydants, les agents mouillants, les antiagglomérants, les dispersants, les émulsionnants, les agents modifiants du goût, les agents de pénétrations, de solubilisation. Les techniques de formulation et d'administration des médicaments et compositions pharmaceutiques sont bien connues dans la technique ici considérée, l'Homme du Métier pouvant notamment se référer à l'ouvrage Remington's Pharmaceutical Sciences, dernière édition.

Selon l'invention, la composition peut être avantageusement administrée par voie orale, par injection en intraveineuse.

Avantageusement, la composition selon l'invention est adaptée pour être administrée par voie orale ou intraveineuse à une dose sensiblement égale ou supérieure à 40 mg/kg/24h et sensiblement égale ou inférieure à 200 mg/kg/24h en une ou plusieurs prises à un mammifère présentant un tel besoin.

La composition selon l'invention peut avantageusement être utilisée dans la prévention et/ou le traitement des infections par un agent pathogène possédant une enveloppe bicouche lipidique, et notamment le VIH, dans la prévention et/ou le traitement du syndrome d'immunodéficience acquise, des maladies opportunistes, notamment les infections à candidoses, la tuberculose, les infections à cytomégalovirus (CMV), à cryptosporidiose, à isosporidiose, à mycobactéries atypiques, la septicémie à salmonella non thyphi récurrente, les ulcères chroniques, les infections bronchiques, pulmonaires ou oesophagiennes, la coccidioidomycose disséminée ou extra-pulmonaire, l'histoplasmose disséminée ou extra-pulmonaire, la cryptococcose extra-pulmonaire, la pneumonie à pneumocystis carinii, la leucoencéphalopathie multifocale progréssive, la toxoplasmose cérébrale.

A titre d'exemples, la composition selon l'invention peut être utilisée pour réduire ou éradiquer le réservoir d'agents pathogènes au niveau du tube digestif, les nombreuses anomalies immunologiques et architecturales au cours de l'infection par des agents pathogènes, notamment le VIH, pour restaurer une immunité muqueuse compétente, en particulier les lymphocytes T.

Le mode d'action de la composition de l'invention n'est pas totalement élucidé. Il est plus que probable qu'elle agisse simultanément sur différents mécanismes en déstructurant, restructurant la composition lipidique des cellules, des agents infectieux, notamment membranaire, en changeant la conformation des protéines qui s'y trouvent, et empêcherait de ce fait l'action des protéines de liaison, de cofacteurs d'entrée, de récepteur alternatif ou co-récepteur alternatif, l'adsorption, l'endocytose, in fine la pénétration des agents pathogènes dans les cellules et l'activation des voies de signalisation cellulaire impliquées dans de nombreux processus physiopathologiques.

Cette modification de la composition lipidique membranaire pourrait aussi diminuer voir inhiber l'affinité, l'assemblage, le bourgeonnement et la maturation des particules virales (certaines protéines virales telles que nef, vif, vpr) au niveau des zones rafts ou micro-domaines membranaires.

D'autre part, cette composition selon l'invention pourrait également être utilisée dans la prévention et/ou le traitement des infections par des virus non enveloppés.

Ainsi, la composition selon l'invention inhiberait la formation, la surexpression et/ou le dysfonctionnement de certains sphingolipides, notamment les glycosphingolipides, la microvésiculation membranaire, la surexpression des récepteurs des chimiokines (CXCR4 et/ou CCR5), la sécrétion des cytokines pro-inflammatoire (le TNF alpha, l'IL-lb, l'IL-6, l'IL-8), la réplication virale, l'inflammation chronique et l'hyperactivation immunitaire systémique, elle préserverait une immunité muqueuse compétente, notamment les cellules immunitaires ciblées par le VIH, en particulier les lymphocytes CD4+, diminuerait ou inhiberait les anomalies de l'architecture du tissu lymphoïde associé au tube digestif (GALT).

Elle peut être utilisée dans la prévention et/ou le traitement des troubles du syndrome métabolique, des maladies cardiovasculaires, de l'accident vasculaire cérébral, des maladies artérielles périphériques, des thromboses veineuses profondes, des maladies pulmonaires, notamment de l'embolie pulmonaire, des maladies auto-immunes, hépatiques, rénales, des maladies neurodégénératives, les complications au niveau du système nerveux central (neuroAIDS).

Cette composition peut aussi être utilisée dans la prévention et/ou le traitement des cancers, notamment les cancers classant sida choisis parmi le sarcome de Kaposi, le lymphome de burkitt, le lymphome immunoblastique, le lymphome cérébral primitif, les lymphomes malins non hodgkiniens (LMNH), et les cancers non classant sida choisi parmi le cancer de la bouche, le cancer de l'estomac, le cancer du côlon, en particulier le cancer invasif du côlon ou colorectal, le cancer du rectum, le cancer anal, le cancer du foie, le carcinome hépatocellulaire, le cancer de la vésicule biliaire, le cancer du pancréas, le cancer du poumon, en particulier l'adénocarcinome du poumon, la leucémie sous la forme chronique ou aigüe, le myélome multiple, le lymphome de Hodgkin, les tumeurs de l'encéphale et d'autres à localisation au niveau du système nerveux, le cancer de la vessie, le cancer de l'ovaire, le cancer de l'utérus, le cancer du rein, le cancer de la prostate et le cancer du sein, les tumeurs osseuses.

Elle inhiberait à cet effet, la prolifération cellulaire, l'angiogenèse, la métastase et provoquerait l'apoptose des cellules cancéreuses. Elle réduirait aussi les états inflammatoires et consoliderait par conséquent un terrain favorable à optimiser la santé.

La présente invention concerne également une préparation pharmaceutique qui comprend la composition selon l'invention, et, en outre, en mélange ou conditionné séparément, au moins un agent anti-diabétique et/ou hypolipémiant, un agent anti-cancéreux, un agent immunostimulant, un agent antiviral pour leur utilisation dans le traitement thérapeutique du syndrome d'immunodéficience acquise, d'une pathologie tumorale et des troubles du syndrome métabolique, de manière simultanée, séquencée ou espacée dans le temps.

A titre d'exemple, l'agent antidiabétique peut être choisi parmi les biguanides, les sulfamides hypoglycémiants et les glinides, les inhibiteurs de l'alpha-glucosidase, les incrétines dont le GLP-1, l'insuline, l'agent hypolipémiant peut être choisi parmi les statines, les fibrates, l'Ezétimibe, l'acide nicotinique, la cholestyramine, l'agent anticancéreux est choisi parmi les anti-métabolites (méthotrexate, capécitabine, 5-fluorouracile), les agents alkylants (cisplatine, mitomycine c, busulfan), les molécules ayant une action sur le fuseau mitotique (vinblastine, vincristine, doxétaxel), les inhibiteurs de la tyrosine kinase (afatinib, erlotinib, sunitinib), les inhibiteurs de la serine thréonine kinase (vemurafenib, everolimus, temsirolimus), les agents agissant sur la topo-isomérase (daunorubicine, doxorubicine, étoposide), les inhibiteurs du protéasome, les inhibiteurs de la DNA méthyltransférase, les inhibiteurs de l'histone déacétylase, les immunomodulateurs (les interférons, les corticoïdes, le talimogène), les anticorps monoclonaux (cetuximab, gemtuzumab, rituximab), certains virus génétiquement modifiés qui ciblent préférentiellement les cellules cancéreuses, la curcumine, le gluthation, la vitamine c et leurs mélanges, et notamment le mélange de deux desdits agents anti-cancéreux, les agents radioactifs utilisables en curiethérapie et/ou les métabolites actifs injectables ou ingérables, l'agent antiviral peut être choisi parmi l'abacavir, abacavir + amphiprine, abacavir + lamivudine +zidovudine, amprénavir, atazanavir, AZT, capravirine, darunavir, ddc, ddl, ddl (enrobage entérosoluble), delavirdine, dolutégravir, doravirine, éfavirenz, éfavirenz + emtricitabine + ténofovir DF, 4-éthynyl-2-fluoro-2'-désoxyadénosine, elvitégravir, emtricitabine + ténofovir DF, emvirine, enfuvirtide, enrobage entériqueà base de didanosine, étravirine, fosamprénavir calcium, indinavir, lamivudine, lamivudine + zidovudine, lopinavir, lopinavir + ritonavir, maraviroc, nelfinavir, névirapine, PPL-100, raltégravir, rilpivirine, ritonavir, saquinavir, stavudine, tipranavir ou vicriviroc.

La présente invention concerne également une préparation pharmaceutique qui comprend en combinaison le béta-élémène et/ou le d-limonene, le lupéol et/ou le béta-sitostérol, l'allicine et/ou l'ajoène, le cinnamaldéhyde et éventuellement le kaempférol.

La présente invention concerne également une préparation pharmaceutique qui comprend en combinaison le béta-élémène et/ou le d-limonene, le lupéol ou un de ses sels pharmaceutiquement acceptables et/ou le béta-sitostérol, l'allicine et/ou l'ajoène, le cinnamaldéhyde et éventuellement le kaempférol.

La présente invention concerne également un complément alimentaire qui comprend en combinaison le béta-élémène et/ou le d-limonene, le lupéol et/ou béta-sitostérol, l'allicine et/ou l'ajoène, le cinnamaldéhyde et éventuellement le kaempférol.

### [Définitions]

Les termes « traitement thérapeutique » font référence au traitement curatif et au traitement prophylactique ; au sens de la présente invention, un traitement thérapeutique permet de restaurer au moins partiellement, de corriger au moins partiellement ou de modifier au moins partiellement des fonctions physiologiques en exerçant une action pharmacologique, immunologique ou métabolique.

Le terme « inflammation » fait référence à une réaction du système immunitaire stéréotypée du corps à une agression externe (infection, trauma, brûlure, allergie, etc) ou interne (cellules cancéreuses). Le caractère chronique de cette inflammation a pour but de maintenir le (ou les) facteur d'agression.

Le terme « patient » fait référence à un mammifère animal ou humain. La composition selon l'invention peut également être utilisée en médecine vétérinaire.

Au sens de la présente invention, un « agent anti-cancéreux » est un élément qui présente au moins in vitro une action contre les cellules cancéreuses, indépendamment de son mécanisme d'action. Par « action » on entend, au sens de la présente invention la destruction ou la modification au moins partielle des cellules cancéreuses qui permet notamment de limiter la prolifération des cellules cancéreuses et/ou leur propagation.

Le terme « infection virale » fait référence à une entité biologique que ce soit le virus de l'hépatite B (VHB), le virus de l'hépatite C (VHC), le virus de l'immunodéficience humaine (VIH), le virus de l'herpes (HSV, herpes simplex virus), le cytomégalovirus (CMV), nécessitant un hôte, le plus souvent une cellule, dont il utilise les constituants pour se multiplier.

Le terme « maladie opportuniste » en médecine humaine ou vétérinaire fait référence à une maladie due à des germes habituellement peu agressifs mais qui sont susceptibles de provoquer de graves complications en affectant des personnes ayant un système immunitaire très affaibli, comme les personnes subissant une greffe d'organe, une chimiothérapie ou atteintes du SIDA.

Les termes « patients atteints de diabète » font référence aux patients atteints de diabète de type 1, les patients atteints de diabète de type 2, les patientes atteintes de diabète gestationnel, les patients atteints de diabète insipide et les patients atteints de diabète rénal.

Le terme « dyslipidémie » fait référence aux hyperlipidémies et aux hypolipidémies déterminées en fonction des critères en vigueur.

Au sens de la présente invention, un « complément alimentaire » est une denrée alimentaire dont le but est de compléter le régime alimentaire normal et qui constitue une source concentrée de nutriments ou d'autres substances ayant un effet nutritionnel ou physiologique seuls ou combinés.

S'agissant des agents cités, les termes utilisés englobent, sauf indications contraires, les isomères de constitution, les stéréo-isomères de conformation, les énantiomères et les diastéréo- isomères du composé chimique considéré.

S'agissant du d-limonène dans la composition selon l'invention, le terme englobe sauf indications contraires, ses dérivés, notamment l'acide périllique, l'acide dihydropérillique, l'alcool périllylique, limonène 1,2-diol et leurs mélanges.

S'agissant du lupéol dans la composition selon l'invention, le terme englobe sauf indications contraires, un de ses sels pharmaceutiquement acceptables (acétate de lupéol, toluate de lupéol, palmitate de lupéol, stéréate de lupéol, cinnamate de lupéol, lupéol-m-chlorobenzoate, lupéol-p-chlorobenzoate), ses dérivés, notamment le stigmastérol, le campestérol, le bétulinol, l'acide bétulinique, l'acide 3-O-(3',3'-dimethylsuccinyl)-bétulinique, l'acide 7β-(4-hydroxybenzoyloxy)-bétulinique et leurs mélanges.

S'agissant du cinnamaldéhyde (CA) dans la composition selon l'invention, le terme englobe sauf indications contraires, ses dérivés, notamment l'acide hydroxycinnamique, le 2-hydroxycinnamaldehyde (HCA), 2'-benzoyloxycinnalmaldéhyde (BCA), le cinnamtannin B1, les dimères de formation, notamment HCA-HCA, BCA-BCA, CA-CA et leurs mélanges.

S'agissant de l'allicine dans la composition selon l'invention, le terme englobe sauf indications contraires, l'alliine, ses dérivés notamment le sulfide de diallyle, le disulfide de diallyle, trisulfure de diallyle, le tétrasulfure de diallyle, le trisulfure de méthyle, le méthyle sulfure d'allyle, l'ajoène et leurs mélanges.

S'agissant du kaempférol dans la composition selon l'invention, le terme englobe sauf indications contraires, le kaempférol-3,7-O-α-1-dirhamnoside et leurs mélanges.

### [Quelques exemples]

Le pourcentage des compositions ci-dessous est un pourcentage en masse par rapport à la masse totale des ingrédients actifs. Exemples non exhaustifs
Composition 1a : d-limonène (20%), lupéol (20%), b-sitostérol (20%), cinnamaldéhyde (20%) et l'allicine (20%).
Composition 1b : béta-élémène (20%), lupéol (20%), b-sitostérol (20%), cinnamaldéhyde (20%) et l'allicine (20%).
Composition 2 : d-limonène (20%), béta-élémène (20%), lupéol (20%), b-sitostérol (20%), cinnamaldéhyde (10%) et l'allicine (10%).
Composition 3a : d-limonène (30%), lupéol (30%), b-sitostérol (10%), cinnamaldéhyde (10%) et l'allicine (20%).
Composition 3b : béta-élémène (30%), lupéol (30%), b-sitostérol (10%), cinnamaldéhyde (10%) et l'allicine (20%).
Composition 4a : d-limonène (20%), lupéol (30%), b-sitostérol (20%), cinnamaldéhyde (20%) et l'allicine (10%).
Composition 4b : béta-élémène (20%), lupéol (30%), b-sitostérol (20%), cinnamaldéhyde (20%) et l'allicine (10%).
Composition 5a : d-limonène (10%), lupéol (10%), b-sitostérol (20%), cinnamaldéhyde (20%) d'allicine (20%) et l'ajoène (20%).
Composition 5b : béta-élémène (10%), lupéol (10%), b-sitostérol (20%), cinnamaldéhyde (30%), l'allicine (20%) et l'ajoène (20%).
Composition 6a : d-limonène (20%), béta-élémène (20%), lupéol (10%), b-sitostérol (20%), cinnamaldéhyde (20%) et l'allicine (10%).
Composition 6b : d-limonène (20%), béta-élémène (20%), lupéol (10%), b-sitostérol (20%), cinnamaldéhyde (20%) et l'ajoène (10%).
Composition 7a : d-limonène (10%), lupéol (20%), b-sitostérol (30%), cinnamaldéhyde (30%) et l'allicine (10%).
Composition 7b : béta-élémène (10%), lupéol (20%), b-sitostérol (30%), cinnamaldéhyde (30%) et l'allicine (10%).

### [Résultats expérimentaux]

Pour examiner l'activité antivirale de cette composition pharmaceutique, les cellules mononucléaires du sang périphérique (CMSP) ont été activées par un mitogène, la phytohémagglutinine, puis infectées par des souches à tropisme lymphocytaire. Toutes ces cellules ont été maintenues à 37°C sous 5% de CO2 dans du RPMI 1640 contenant 10% (vol/vol) de sérum de veau foetal, 2 mM de I-glutamine, une solution de trois antibiotiques (pénicilline, streptomycine, néomycine). Sept jours après l'infection, les surnageants de culture ont été collectés et congelés à -20°C jusqu'à la mesure de la réplication virale via la quantification de l'activité enzymatique de la transcriptase inverse.

En parallèle, au septième jour de la culture, la cytotoxicité des molécules ont été évaluée vis à vis des CMSP activées par la phytohémagglutinine, mais non infectées. Ces éventuels effets vis à vis de la viabilité cellulaire ont été mesurés à l'aide d'un test colorimétrique au sel de méthyl tétrazolium.

Les pourcentages d'inhibition de l'activité enzymatique de la transcriptase inverse et de la viabilité cellulaire seront calculés afin de déterminer respectivement les concentrations effectrices 50, 70 et 90% (CE50, CE70 et CE90) et les concentrations cytotoxiques (CC50, CC70 et CC90) à partir d'une courbe effet-dose à quatre paramètres. Les indices de sélectivité (IS) ont été calculés selon le rapport CC50/CE50.

Dans les compositions à tester, il a été observé une diminution de la fixation du virus aux CMSP, une limitation de la réplication virale. Le mode d'action anti-VIH pourrait être attribué à l'inhibition des événements précoces à la réplication virale, en particulier l'adsorption du virus. Cette composition pharmaceutique pourrait aussi inhiber l'inflammation chronique observée chez les personnes vivants avec le VIH.

Cette composition pharmaceutique peut donc à juste titre être utilisée dans la prévention et le traitement du syndrome d'immunodéficience acquise, des maladies opportunistes liée à l'immunodéficience, des troubles du syndrome métabolique et du cancer.

## Revendications

1. Composition pharmaceutique **caractérisée en ce qu'**elle comprend en combinaison le béta-élémène, le d-limonene, le lupéol ou un de ses sels pharmaceutiquement acceptables, le béta-sitostérol, l'allicine, l'ajoène, le cinnamaldéhyde et éventuellement le kaempférol.

2. Composition pharmaceutique selon la revendication 1, **caractérisée en ce qu'**elle comprend, en outre un mélange béta-sitostérol et de cinnamaldéhyde ou de béta-sitostérol et de d'allicine et/ou d'ajoène ou un mélange de béta-sitostérol et de kaempférol.

3. Composition pharmaceutique selon l'une quelconque des revendications précédentes, **caractérisée en ce qu'**elle comprend en pourcentage massique de la masse totale des ingrédients actifs, un pourcentage de béta-élémène sensiblement égal ou supérieur à 10% et sensiblement égal ou inférieur à 50%, et notamment sensiblement égal ou supérieur à 20% et sensiblement égal ou inférieur à 30%, un pourcentage massique de d-limonene sensiblement égal ou supérieur à 10% et sensiblement égal ou inférieur à 55%, notamment sensiblement égal ou supérieur 20% et sensiblement égal ou inférieur à 40%, un pourcentage de lupéol ou un de ses sels pharmaceutiquement acceptables sensiblement égal ou supérieur 10% et sensiblement égal ou inférieur à 50%, et notamment sensiblement égal ou supérieur à 20% et sensiblement égal ou inférieur à 30%, un pourcentage de beta-sitostérol sensiblement égal ou supérieur à 10% et sensiblement égal ou inférieur à 50%, et notamment sensiblement égal ou supérieur à 20% et sensiblement égal ou inférieur à 30%, un pourcentage de cinnamaldéhyde sensiblement égal ou supérieur à 10% et sensiblement égal ou inférieur à 50%, et notamment sensiblement égal ou supérieur à 20% et sensiblement égal ou inférieur à 40%, un pourcentage d'allicine, lorsque ladite composition contient cet ingrédient, sensiblement égal ou supérieur à 10% et sensiblement égal ou inférieur à 50%, et notamment sensiblement égal ou supérieur à 20% et sensiblement égal ou inférieur à 40%, un pourcentage d'ajoène lorsque ladite composition contient cet ingrédient, sensiblement égal ou supérieur à 10% et sensiblement égal ou inférieur à 50%, et notamment sensiblement égal ou supérieur à 20% et sensiblement égal ou inférieur à 40%, un pourcentage de kaempférol lorsque ladite composition contient cet ingrédient, sensiblement égal ou supérieur à 10% et sensiblement égal ou inférieur à 50%, et notamment sensiblement égal ou supérieur à 20% et sensiblement égal ou inférieur à 40%.

4. Composition pharmaceutique selon l'une quelconque des revendications précédentes utilisable en tant que médicament, et plus particulièrement utilisable pour réduire ou éradiquer le réservoir d'agents pathogènes au niveau du tube digestif, les nombreuses anomalies immunologiques et architecturales au cours de l'infection par des agents pathogènes et notamment le VIH, pour restaurer une immunité muqueuse compétente, en particulier les lymphocytes T.

5. Composition pharmaceutique selon la revendication 4, pour utilisation dans la prevention et/ou le traitement de l'inflammation chronique, l'hyperactivation immunitaire systémique, du syndrome de cachexie.

6. Composition pharmaceutique selon l'une quelconque des revendications précédentes pour utilisation dans la prévention et/ou le traitement des infections, et notamment le VIH, dans la prévention et/ou le traitement du syndrome d'immunodéficience acquise, des maladies opportunistes, notamment les infections à candidoses, la tuberculose, les infections à cytomégalovirus (CMV), à cryptosporidiose, à isosporidiose, à mycobactéries atypiques, la septicémie à salmonella non thyphi recurrente, les ulceres chroniques, les infections bronchiques, pulmonaires ou oesophagiennes, la coccidioidomycose disséminée ou extra-pulmonaire, l'histoplasmose disséminée ou extra-pulmonaire, la cryptococcose extra-pulmonaire, la pneumonie à pneumocystis carinii, la leucoencephalopathie multifocale progressive, la toxoplasmose cérébrale.

7. Composition pharmaceutique selon l'une quelconque des revendications precedentes, pour utilisation dans la prevention et/ou le traitement du diabete, des troubles du syndrome metabolique, des maladies cardiovasculaires, de l'accident vasculaire cerebral, des maladies arterielles peripheriques, des thromboses veineuses profondes, des maladies pulmonaires, notamment de l'embolie pulmonaire, des maladies auto-immunes, hepatiques, renales, des maladies neurodegeneratives, les complications au niveau du systeme nerveux central (neuroAIDS).

8. Composition pharmaceutique selon l'une quelconque des revendications précédentes, pour utilisation dans la prévention et/ou le traitement des cancers, notamment les cancers classant sida choisis parmi le sarcome de Kaposi, le lymphome de burkitt, le lymphome immunoblastique, le lymphome cérébral primitif, les lymphomes malins non hodgkiniens (LMNH), et les cancers non classant sida choisi parmi le cancer de la bouche, le cancer de l'estomac, le cancer du côlon, en particulier le cancer invasif du côlon ou colorectal, le cancer du rectum, le cancer anal, le cancer du foie, le carcinome hépatocellulaire, le cancer de la vésicule biliaire, le cancer du pancréas, le cancer du poumon, en particulier l'adénocarcinome du poumon, la leucémie sous la forme chronique ou aigüe, le myélome multiple, le lymphome de Hodgkin, les tumeurs de l'encéphale et d'autres à localisation au niveau du système nerveux, le cancer de la vessie, le cancer de l'ovaire, le cancer de l'utérus, le cancer du rein, le cancer de la prostate et le cancer du sein, les tumeurs osseuses.

9. Préparation pharmaceutique **caractérisée en ce qu'**elle comprend la composition selon l'une quelconque des revendications précédentes et, en outre, en mélange ou conditionné séparément, au moins un agent anti-diabétique et/ou hypolipémiant, un agent anti-cancéreux, un agent immunostimulant, un agent antiviral pour leur utilisation dans le traitement thérapeutique du syndrome d'immunodéficience acquise, d'une pathologie tumorale et des troubles du syndrome métabolique.

10. Préparation pharmaceutique selon la revendication 9, **caractérisée en ce que** ledit agent antidiabétique peut être choisi parmi les biguanides, les sulfamides hypoglycémiants et les glinides, les inhibiteurs de l'alpha-glucosidase, les incrétines dont le GLP-1, l'insuline, l'agent hypolipémiant peut être choisi parmi les statines, les fibrates, l'Ezétimibe, l'acide nicotinique, la cholestyramine, l'agent anticancéreux est choisi parmi les anti-métabolites (méthotrexate, capécitabine, 5-fluorouracile), les agents alkylants (cisplatine, mitomycine c, busulfan), les molécules ayant une action sur le fuseau mitotique (vinblastine, vincristine, doxétaxel), les inhibiteurs de la tyrosine kinase (afatinib, erlotinib, sunitinib), les inhibiteurs de la serine thréonine kinase (vemurafenib, everolimus, temsirolimus), les agents agissant sur la topo-isomérase (daunorubicine, doxorubicine, étoposide), les inhibiteurs du protéasome, les inhibiteurs de la DNA méthyltransférase, les inhibiteurs de l'histone déacétylase, les immunomodulateurs (les interférons, les corticoïdes, le talimogène), les anticorps monoclonaux (cetuximab, gemtuzumab, rituximab), certains virus génétiquement modifiés qui ciblent préférentiellement les cellules cancéreuses, la curcumine, le gluthation, la vitamine c et leurs mélanges, et notamment le mélange de deux desdits agents anti-cancéreux, les agents radioactifs utilisables en curiethérapie et/ou les métabolites actifs injectables ou ingérables, l'agent antiviral peut être choisi parmi l'abacavir, abacavir + amphiprine, abacavir + lamivudine +zidovudine, amprénavir, atazanavir, AZT, capravirine, darunavir, ddc, ddl, ddl (enrobage entérosoluble), delavirdine, dolutégravir, doravirine, éfavirenz, éfavirenz + emtricitabine + ténofovir DF, 4-éthynyl-2-fluoro-2'-désoxyadénosine, elvitégravir, emtricitabine + ténofovir DF, emvirine, enfuvirtide, enrobage entériqueà base de didanosine, étravirine, fosamprénavir calcium, indinavir, lamivudine, lamivudine + zidovudine, lopinavir, lopinavir + ritonavir, maraviroc, nelfinavir, névirapine, PPL-100, raltégravir, rilpivirine, ritonavir, saquinavir, stavudine, tipranavir ou vicriviroc.
